(19) Europäisches Patentamt
European Patent Office
Office européen des brevets

(11) **EP 2 853 933 B1**

(12) **EUROPÄISCHE PATENTSCHRIFT**

(45) Veröffentlichungstag und Bekanntmachung des Hinweises auf die Patenterteilung:
**22.05.2019 Patentblatt 2019/21**

(51) Int Cl.:
*G02B 21/02* *(2006.01)* *G02B 21/22* *(2006.01)*
*G02B 21/00* *(2006.01)*

(21) Anmeldenummer: **14184905.9**

(22) Anmeldetag: **16.09.2014**

(54) **Optisches Abbildungssystem**

Optical imaging system

Système d'imagerie optique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.09.2013 DE 102013219379**

(43) Veröffentlichungstag der Anmeldung:
**01.04.2015 Patentblatt 2015/14**

(73) Patentinhaber: **Carl Zeiss Meditec AG**
**07745 Jena (DE)**

(72) Erfinder:
• **Merz, Franz**
**73433 Aalen (DE)**
• **Högele, Artur**
**73447 Oberkochen (DE)**

(74) Vertreter: **Carl Zeiss AG - Patentabteilung**
**Carl-Zeiss-Strasse 22**
**73447 Oberkochen (DE)**

(56) Entgegenhaltungen:
**EP-A1- 1 326 117 CH-A5- 663 284**
**DE-A1-102010 018 123**

**Beschreibung**

Die Erfindung betrifft ein optisches Abbildungssystem, insbesondere ein Mikroskop, zur Erzeugung eines Bildes einer Objektebene mit einem Linsensystem, das ein Hauptobjektiv und eine Reduzieroptik zwischen dem Hauptobjektiv und der Objektebene umfasst und das entlang einer optischen Achse ausgerichtet ist. Die Reduzieroptik weist eine erste Linse mit einer positiven Brechkraft und eine zweite Linse mit einer negativen Brechkraft auf. Durch das Linsensystem ist eine objektseitige erste Hauptebene und eine bildseitige zweite Hauptebene definiert. Durch das optische Abbildungssystem ist ein Beobachtungsstrahlengang definiert, der derart durch das Linsensystem geführt ist, dass der Beobachtungsstrahlengang in der ersten Hauptebene und in der zweiten Hauptebene jeweils einen Abstand zur optischen Achse des Linsensystems aufweist.

Bei der Beobachtung eines Objektes durch ein optisches Abbildungssystem, insbesondere ein Stereo-Operationsmikroskop, kann zwischen dem Hauptobjektiv des optischen Abbildungssystems und dem zu beobachtenden Objekt, beispielsweise ein Auge, eine Weitwinkel-Optik in den Strahlengang eingebracht werden. Dies ermöglicht eine Beobachtung des Augenhintergrundes. Zusätzlich zu dieser Weitwinkel-Optik kann eine Reduzieroptik in Strahlengang zwischen der Weitwinkel-Optik und dem Hauptobjektiv des optischen Abbildungssystems eingeschwenkt werden, um die Adaption der Weitwinkel-Optik an ein optisches Abbildungssystem, beispielsweise ein Operationsmikroskop, zu ermöglichen.

Aus der EP 1 227 355 B1 ist ein Mikroskop zur Weitwinkel-Betrachtung für Augenoperationen bekannt, das durch wahlweise zuschaltbare Optiken ermöglicht, von dem Augenhintergrund ein Bild zu erzeugen. Das Mikroskop umfasst ein Linsensystem, das ein Hauptobjektiv und vor dem Hauptobjektiv angeordnete Linsen aufweist.

Dieses Mikroskop hat den Nachteil, dass die Abbildungsqualität bei der Weitwinkel-Betrachtung nicht optimal ist. Durch den geringen Abstand des Hauptobjektivs zum zu beobachtenden Objekt ergeben sich für eine in den Strahlengang zuschaltbare Weitwinkeloptik sehr kleine Brennweiten. Durch eine weitere, in den Strahlengang einbringbare Reduzieroptik kann eine Anpassung der Weitwinkeloptik an das Mikroskop erfolgen, jedoch mit dem Nachteil einer reduzierten Abbildungsqualität.

Zusätzlich zu den optischen Nachteilen weist das optische Abbildungssystem gemäß EP 1 227 355 B1 eine relativ große Baulänge auf. Um den Anwender, beispielsweise einen Chirurgen, bei seiner Arbeit mit dem optischen Abbildungssystem am Objekt, beispielsweise einem Patientenauge, nicht unnötig zu behindern, sollte eine Reduzieroptik nur eine geringe Baulänge aufweisen und dazu möglichst nahe am Hauptobjektiv angeordnet sein.

Weiterer Stand der Technik ist aus den Druckschriften EP 1 326 117 A1, CH 663 284 A5 und DE 10 2010 018123 A1 bekannt. Aufgabe der Erfindung ist es, ein optisches Abbildungssystem bereitzustellen, bei dem unter Verwendung einer Reduzieroptik vor einem Hauptobjektiv eine sehr gute Abbildungsqualität erreicht wird. Ferner ist es eine Aufgabe, ein Abbildungssystem mit einer geringen Baulänge bereitzustellen.

Die Aufgabe gemäß der Erfindung wird durch ein optisches Abbildungssystem entsprechend Anspruch 1 gelöst.

Erfindungsgemäß wird die Aufgabe dadurch gelöst, dass die erste Linse der Reduzieroptik aus einem ersten Material gefertigt ist, das eine erste Abbezahl aufweist, und dass die zweite Linse der Reduzieroptik aus einem zweiten Material gefertigt ist, das eine zweite Abbezahl aufweist, wobei die erste Abbezahl größer ist als die zweite Abbezahl. Dabei ist das Linsensystem derart ausgestaltet, dass für alle Wellenlängen im Wellenlängenbereich $\lambda$ von 480nm $\leq \lambda$ $\leq$ 660nm und für eine Hauptwellenlänge e = 546nm folgende Beziehung erfüllt ist:

$$\left| \arctan\left( \frac{B}{fe' + (fe \cdot fe' / (f_\lambda - fe))} \right) \right| < 0.5',$$

wobei gilt:

B      = Abstand eines Beobachtungsstrahlengangs zur optischen Achse in der ersten Hauptebene H;
fe     = objektseitige Brennweite für die Hauptwellenlänge e bezüglich der ersten Hauptebene H;
$f_\lambda$   = objektseitige Brennweite für die Wellenlänge $\lambda$ bezüglich der ersten Hauptebene H;
fe' =    bildseitige Brennweite für die Hauptwellenlänge e bezüglich der zweiten Hauptebene H'.

Die Einheit des Terms "0.5' " ist Winkelminuten.
Ist das Linsensystem derart ausgestaltet, dass für alle Wellenlängen $\lambda$ zwischen 480nm und 660nm und eine Hauptwellenlänge e = 546nm folgende Bedingung erfüllt ist,

$$\left| \arctan\left( \frac{B}{fe' + (fe \cdot fe' / (f_\lambda - fe))} \right) \right| < 0.5',$$

so ist die Abbildungsqualität für einen Beobachtungsstrahlengang so gut, dass kontrastmindernde und störende Bildfehler korrigiert sind, so dass ein gleichbleibender guter Kontrast der Abbildung und eine gleichbleibende gute Bildqualität über den gesamten Wellenlängenbereich $\lambda$ erreicht werden.

In einer Ausgestaltung der Erfindung sind das erste Material und das zweite Material derart gewählt, dass eine Differenz der ersten Abbezahl und der zweiten Abbezahl zwischen 16 und 22 liegt.

Bei der Gestaltung des Linsensystems hat es sich als besonders vorteilhaft erwiesen, das erste Material der Linse mit der positiven Brechkraft und das zweite Material der Linse mit der negativen Brechkraft so zu wählen, dass die Differenz der Abbezahlen der beiden Materialien zwischen 16 und 22 liegt. Damit kann die beschriebene Bedingung gut erfüllt werden und es ergibt sich vorteilhaft ein sehr guter Kontrast über den gesamten Wellenlängenbereich bei einer geringen chromatischen Winkelabweichung. Besonders vorteilhaft wird die Linse mit der positiven Brechkraft aus einem Material mit hoher Abbezahl und für die Linse mit der negativen Brechkraft aus einem Material mit niedriger Abbezahl gebildet, wobei die Differenz der Abbezahlen zwischen 16 und 22 liegt.

In einer weiteren Ausgestaltung der Erfindung sind das erste Material und das zweite Material derart gewählt, dass eine erste Brechzahl des ersten Materials größer ist als 1,6 und eine zweite Brechzahl des zweiten Materials größer ist als 1,6.

Durch die Verwendung von Materialien mit hohen Brechzahlen, d. h. größer als 1.6, für die erste und zweite Linse der Reduzieroptik kann die obige Bedingung gut erfüllt werden. Damit ergibt sich vorteilhaft ein gut korrigiertes Bild mit sehr gutem Kontrast und einer geringen chromatischen Winkelabweichung.

In einer weiteren Ausgestaltung der Erfindung ist die erste Linse fest angeordnet und die zweite Linse ist verschiebbar in Richtung der optischen Achse angeordnet.

Die zu beobachtende Objektebene kann in der Entfernung von dem Hauptobjektiv variieren. Damit kann es notwendig sein, den Fokus der optischen Beobachtungseinrichtung an die geänderte Objektebene anzupassen. Damit die Fokuseinstellung des Mikroskops unverändert bleiben kann, ist es vorteilhaft, wenn die Reduzieroptik die Möglichkeit einer Fokussierung bietet. Diese Fokussiermöglichkeit lässt sich relativ einfach dadurch erreichen, indem die erste Linse der Reduzieroptik fest angeordnet ist und die zweite Linse eine Relativbewegung entlang der optischen Achse ausführen kann. Durch die Erfüllung der Bedingung im Anspruch 1 ist über den gesamten Fokussierbereich eine gute Korrektur der chromatischen Winkelabweichung gewährleistet.

In einer weiteren Ausgestaltung der Erfindung ist die zweite Linse fest angeordnet und die erste Linse ist verschiebbar in Richtung der optischen Achse angeordnet.

Die gleichen Vorteile, die in der vorherigen Ausgestaltung beschrieben sind, lassen sich erreichen, wenn die zweite Linse der Reduzieroptik fest angeordnet ist und die erste Linse der Reduzieroptik verschiebbar in Richtung der optischen Achse angeordnet ist.

In einer weiteren Ausgestaltung der Erfindung ist die Reduzieroptik in den Strahlengang vor dem Hauptobjektiv einschwenkbar.

Um dem Anwender eines Mikroskops zu ermöglichen, variabel mit oder ohne Reduzieroptik zu arbeiten, ist es vorteilhaft, wenn die Reduzieroptik einfach in den Strahlengang eingebracht oder aus dem Strahlengang entfernt werden kann. Dadurch kann der Anwender schnell und einfach zwischen zwei Fokusebenen hin- und herschalten, ohne die Fokuseinstellung des Mikroskops verändern zu müssen. Vorteilhaft lässt sich die Reduzieroptik sehr einfach und schnell durch eine Schwenkvorrichtung in den Strahlengang vor dem Hauptobjektiv ein- und ausschwenken.

In einer weiteren Ausgestaltung der Erfindung ist vor der Reduzieroptik ein weiteres optisches Element zur Erzeugung eines Zwischenbildes im Beobachtungsstrahlengang angebracht und das optische Abbildungssystem ist auf das Zwischenbild fokussiert.

Vor der Reduzieroptik kann ein weiteres optisches Element in den Strahlengang eingebracht sein. Die beobachtete Objektebene kann dann eine Zwischenbildebene darstellen, die sich räumlich zwischen dem weiteren optischen Element und der Reduzieroptik im Strahlengang ergibt. Mängel in der Abbildungsqualität, die durch das Einbringen des weiteren optischen Elementes entstehen, lassen sich besonders vorteilhaft durch die Reduzieroptik chromatisch korrigieren, so dass ein sehr gutes, kontrastreiches Bild ohne Bildversatz beobachtet werden kann.

In einer weiteren Ausgestaltung der Erfindung ist das optische Abbildungssystem als ein Stereomikroskop ausgebildet, das einen ersten Beobachtungsstrahlengang und einen zweiten Beobachtungsstrahlengang aufweist, wobei der erste und der zweite Beobachtungsstrahlengang in der ersten Hauptebene H und in der zweiten Hauptebene H' jeweils einen Abstand B zur optischen Achse des Linsensystems aufweisen.

Die Linsen der Reduzieroptik sind üblicherweise rotationssymmetrisch bezüglich der optischen Achse ausgeführt. Dadurch, dass die optische Achse des Hauptobjektivs und die optische Achse der Reduzieroptik identisch sind, ist die optische chromatische Korrektur für alle Beobachtungsstrahlengänge, die in einem Abstand B zur optischen Achse bezüglich der beiden Hauptebenen H und H' verlaufen, gleich gut. Für eine Stereomikroskop ergibt sich dadurch der Vorteil, dass durch eine einzige Reduzieroptik beide Beobachtungsstrahlengänge chromatisch gleich gut korrigiert sind. Dieser Vorteil gilt auch für jeden weiteren Beobachtungsstrahlengang, der in einem Abstand B zur optischen Achse bezüglich der beiden Hauptebenen H und H' geführt ist.

Weitere Vorteile und Merkmale der Erfindung werden in Bezug auf die nachfolgenden Zeichnungen erklärt, in welchen zeigen:

Figur 1   ein erstes Ausführungsbeispiel eines erfindungsgemäßen optischen Abbildungssystems mit einer vor einem Hauptobjektiv angebrachten Reduzieroptik;

Figur 2   eine schematische Darstellung eines Strahlengangs in einem optischen Abbildungssystem gemäß Figur 1;

Figur 3   eine bildseitige chromatische Winkelabweichung bezogen auf eine Wellenlänge zwischen 480nm und 660nm für drei unterschiedliche Brennweiten des optischen Abbildungssystems gemäß Figur 1.

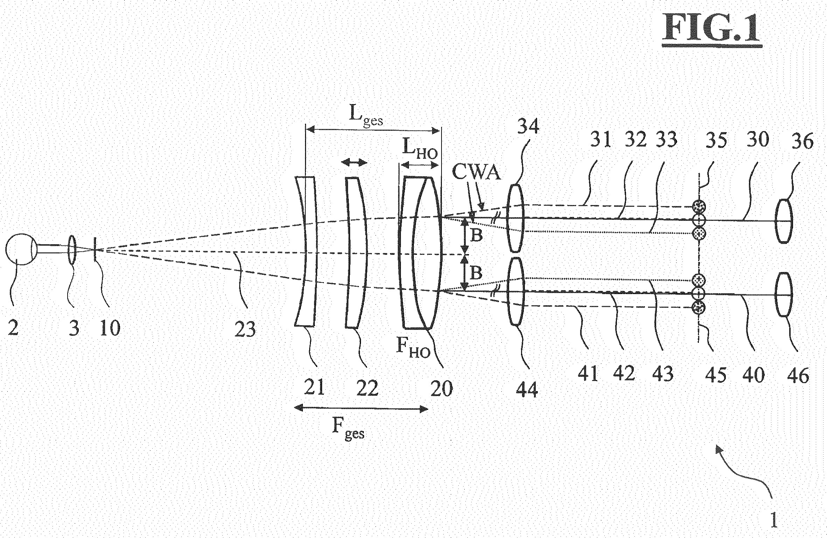

In Figur 1 ist ein Ausführungsbeispiel eines erfindungsgemäßen optischen Abbildungssystems 1 mit einer vor einem Hauptobjektiv 20 angebrachten Reduzieroptik dargestellt.

Das Ausführungsbeispiel zeigt ein optisches Abbildungssystems 1 zur Beobachtung eines Auges 2. Das optische Abbildungssystem 1 ist als stereoskopisches Beobachtungssystem mit einem rechten Beobachtungsstrahlengang 30 und einem linken Beobachtungsstrahlengang 40 ausgelegt und weist ein Hauptobjektiv 20 mit einer optischen Achse 23, eine rechte Tubuslinse 34, eine linke Tubuslinse 44, sowie ein rechtes Okular 36 und ein linkes Okular 46 auf. Es kann weitere, nicht dargestellte, Optik-Elemente umfassen.

Zwischen dem Hauptobjektiv 20 und dem Auge 2 ist ein weiteres optisches Element in Form einer Ophthalmoskopierlupe 3 und eine Reduzieroptik in Form von zwei Linsen in den Strahlengang eingebracht. Eine direkt vor dem Hauptobjektiv 20 angeordnete erste Linse der Reduzieroptik ist als eine Sammellinse 22 ausgeführt und weist eine positive Brechkraft auf. Eine zweite Linse der Reduzieroptik ist als eine Linse mit negativer Brechkraft, als eine Zerstreuungslinse 21 ausgeführt.

Der rechte Beobachtungsstrahlengang 30 und der linke Beobachtungsstrahlengang 40 durchsetzen die Ophthalmoskopierlupe 3 und können sich in einer Bildebene 10 kreuzen. Der von der Bildebene 10 ausgehende rechte Beobachtungsstrahlengang 30 durchdringt die Zerstreuungslinse 21, die Sammellinse 22, das Hauptobjektiv 20, die rechte Tubuslinse 34 und gelangt zu dem rechten Okular 36. Dabei wird in dem rechten Beobachtungsstrahlengang 30 in einer rechten Okular-Zwischenbildebene 35 ein Okularzwischenbild erzeugt, das durch das rechte Okular 36 durch einen Beobachter betrachtet werden kann. Der von der Bildebene 10 ausgehende linke Beobachtungsstrahlengang 40 ist durch die Zerstreuungslinse 21, die Sammellinse 22, das Hauptobjektiv 20, die linke Tubuslinse 44 zu dem linken Okular 46 geführt. Dabei wird in dem linken Beobachtungsstrahlengang in einer linken Okular-Zwischenbildebene 45 ein Okularzwischenbild erzeugt, das durch das linke Okular 46 durch einen Beobachter betrachtet werden kann.

Der rechte Beobachtungsstrahlengang 30 verläuft nach dem Hauptobjektiv 20 parallel zu der optischen Achse 23. Dieser parallele Abstand ist mit B gekennzeichnet. Entsprechend verläuft der linke Beobachtungsstrahlengang 40 nach dem Hauptobjektiv 20 ebenso in einem Abstand B parallel zu der optischen Achse 23. Der parallele Abstand der beiden Beobachtungsstrahlengänge 30, 40 nach dem Hauptobjektiv 20 kann auch als Stereobasis SB bezeichnet werden, wobei die Stereobasis SB einen Wert aufweist, der doppelt so groß ist wie der Abstand B. Ein typischer Zahlenwert für die Stereobasis SB kann 25mm sein.

Typische Werte für die Brennweiten der Ophthalmoskopierlupe 3 sind 60 Dioptrien, 90 Dioptrien oder 120 Dioptrien. Die kurze Brennweite der Ophthalmoskopierlupe 3 kann eine Ursache für auftretende Bildfehler sein, die als spektrale Verkippung der optischen Achsen oder als eine chromatische Winkelabweichung CWA bezeichnet werden.

In Figur 1 sind schematisch die Auswirkungen der chromatischen Winkelabweichung CWA dargestellt. Unter der chromatischen Winkelabweichung CWA ist ein farbabhängiger chromatischer Bildversatz der Okularzwischenbilder, senkrecht zur optischen Achse 23, zu verstehen.

Für den rechten Beobachtungsstrahlengang 30 und den linken Beobachtungsstrahlengang 40 sind, bedingt durch einen symmetrischen Aufbau, jeweils vom Betrag her gleiche Werte für die chromatische Winkelabweichung CWA zu erwarten. Eine chromatische Winkelabweichung kann auch für einen einzelnen Beobachtungsstrahlengang auftreten. Für den rechten Beobachtungsstrahlengang 30 sind chromatische Winkelabweichungen für drei Wellenlängenbereiche in Form von drei Teilstrahlen dargestellt: Ein rechter roter Teilstrahl 31, ein rechter grüner Teilstrahl 32 und ein rechter blauer Teilstrahl 33. Für den linken Beobachtungsstrahlengang 40 ist eine chromatische Winkelabweichung für drei Wellenbereiche in Form eines linken roten Teilstrahls 41, eines linken grünen Teilstrahls 42 und eines linken blauen Teilstrahls 43 dargestellt. Der Bildversatz der rechten Teilstrahlen 31, 32, 33 im rechten Beobachtungsstrahlengang 30 ist in der rechten Okular-Zwischenbildebene 35 durch das rechte Okular 36 sichtbar. Der Bildversatz der linken Teilstrahlen 41, 42, 43 im linken Beobachtungsstrahlengang 40 ist in der linken Okular-Zwischenbildebene 45 durch das linke Okular 46 zu sehen.

Der Bildversatz, bedingt durch die chromatische Winkelabweichung CWA, wird ohne chromatische Korrektur als kontrastmindernd und störend empfunden. Liegt dieser chromatische Bildversatz über der Auflösungsgrenze des Auges, nimmt der Betrachter farbige Doppelbilder wahr. Der grüne Wellenlängenbereich, in Form der grünen Teilstrahlen 32, 42 ist im Zentrum der Okulare dargestellt. Der blaue Bildbereich in Form der blauen Teilstrahlen 33, 43 ist innerhalb des Bereichs zwischen den beiden Okularzentren sichtbar. Der rote Wellenlängenbereich, in Form der roten Teilstrahlen 31,41, ist außerhalb des Bereichs zwischen den Okularzentren sichtbar.

In diesem Ausführungsbeispiel ist die Reduzieroptik, umfassend die Sammellinse 22 und die Zerstreuungslinse 21, sehr nahe an dem Hauptobjektiv 20 angeordnet. Das Hauptobjektiv 20 weist eine Baulänge $L_{HO}$ auf. Als Baulänge $L_{HO}$ wird die Dicke oder Ausdehnung des Hauptobjektivs 20 entlang der der optischen Achse 23 bezeichnet. Eine gesamte Baulänge $L_{ges}$ bezeichnet die größte Ausdehnung des Hauptobjektivs 20 und der Reduzieroptik, umfassend die Zerstreuungslinse 21 und die Sammellinse 22, bezüglich der optischen Achse 23. Weist das Hauptobjektiv 20 beispielsweise eine Baulänge $L_{HO}$ = 1cm auf, so ergibt sich für die gesamte Baulänge $L_{ges}$ < 3,2cm.

Bedingt durch die sehr nahe Anordnung der Reduzieroptik vor dem Hauptobjektiv 20 stellt die Bereitstellung eines kontrastreichen, chromatisch korrigierten Bildbereichs mit sehr guter Abbildungsqualität eine besondere Herausforderung dar.

Es sei noch darauf verwiesen, dass die in diesem Ausführungsbeispiel gezeigte Bildebene 10 als Zwischenbildebene dargestellt ist, in der sich der rechte Beobachtungsstrahlengang 30 und der linke Beobachtungsstrahlengang kreuzen. Die Bildebene 10 kann auch eine andere zu beobachtende Objektebene darstellen. Ebenso kann die Position der Zerstreuungslinse 21 und die Position der Sammellinse 22 vertauscht angeordnet sein.

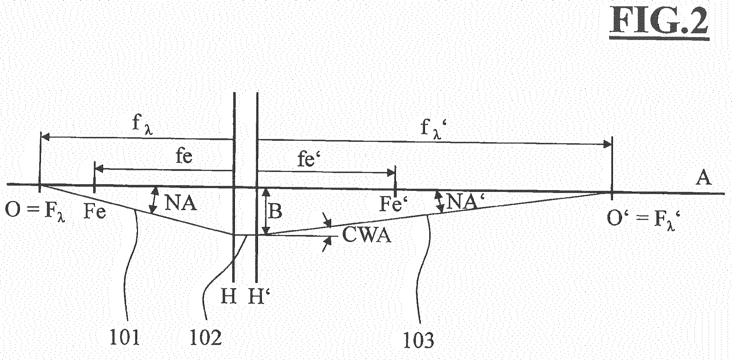

In Figur 2 zeigt eine schematische Darstellung eines Strahlengangs des optischen Abbildungssystems 1 gemäß Figur 1.

Eine optische Achse A ist als dicke horizontale Line gezeichnet. Senkrecht zu der optischen Achse A befindet sich eine erste Hauptebene H als Bezugsebene für Brennweiten oder Abstandsangaben im Objektraum und eine zweite Hauptebene H' als Bezugsebene für den Bildraum. Die beiden Hauptebenen H, H' erlauben es, die Wirkung des komplexen optischen Linsensystems mit der für eine dünne Linse gültigen Gleichung zu beschreiben. Die erste Hauptebene H und die zweite Hauptebene H' sind beide senkrecht zur optischen Achse des Linsensystems definiert und verlaufen damit parallel zueinander. Die beiden Hauptebenen H, H' ersetzen das Hauptobjektiv 20 sowie die Reduzieroptik, umfassend die Zerstreuungslinse 21 und die Sammellinse 22.

Ein Objektpunkt O wird durch das optische Linsensystem zu einem Bildpunkt O' abgebildet. Von dem Objektpunkt O verläuft ein erster Strahl 101 in einem Winkel NA bezüglich der optischen Achse A zu der ersten Hauptebene H. Der Winkel NA wird auch als objektseitige numerische Apertur bezeichnet. Zwischen der ersten Hauptebene H und der zweiten Hauptebene H' verläuft ein zweiter Strahl 102 parallel zu der optischen Achse A. Der zweite Strahl 102 hat in der ersten Hauptebene H und in der zweiten Hauptebene H' jeweils den Abstand B zur optischen Achse A. Bei einem stereoskopischen Abbildungssystem kann B einen Wert aufweisen, der vom Betrag her dem Zahlenwert der halben Stereobasis SB entspricht. Beim Austritt aus der zweiten Hauptebene H' ist ein dritter Strahl 103 zum Bildpunkt 0' gerichtet. Der dritte Strahl 103 schließt einen Winkel CWA bezüglich der optischen Achse A ein. Der Winkel CWA stellt die bildseitige chromatische Winkelabweichung CWA dar. Ist die bildseitige chromatische Winkelabweichung CWA gleich null, dann liegt der Bildpunkt O' im Unendlichen.

Für den Objektraum hat ein objektseitiger Brennpunkt Fe die objektseitige Brennweite fe für eine Hauptwellenlänge e. Im Bildraum hat der bildseitige Brennpunkt Fe' die bildseitige Brennweite fe' für die Hauptwellenlänge e. Für eine Wellenlänge A ergeben sich damit konform eine objektseitige Brennweite $f_\lambda$ für einen objektseitigen Brennpunkt O = $F_\lambda$, sowie eine bildseitige Brennweite $f_\lambda$' für einen bildseitigen Brennpunkt O' = $F_\lambda$'.

Zur Lösung der Aufgabe hat sich herausgestellt, dass die Maßzahl der chromatischen Winkelabweichung CWA eine sehr gute Möglichkeit darstellt, Maßnahmen zur Optimierung der Bildqualität zu bewerten. Sinnvollerweise geht man bei der Berechnung der Optik von einer Hauptwellenlänge e = 546nm aus. Die Hauptwellenlänge e wird auch als Fraunhofer-Linie e bezeichnet und definiert die Hauptwellenlänge im grünen Spektralbereich der Sonne. Um einen Objektpunkt O, beispielsweise die Bildebene 10, kontraststark und mit sehr guter Abbildungsqualität abzubilden, ist es notwendig, eine Optik bereitzustellen, die über den sichtbaren Wellenlängenbereich $\lambda$ zwischen 480nm und 660nm chromatisch korrigiert ist.

Vor dem Hauptobjektiv 20 ist die Reduzieroptik angeordnet, die die aus unterschiedlichen Materialien gefertigte Zerstreuungslinse 21 und Sammellinse 22, umfasst. Zum Erreichen einer guten Bildqualität wird für die Zerstreuungslinse 21 ein Material mit hoher Dispersion, d. h. niedriger Abbezahl gewählt. Die Sammellinse 22 weist ein Material mit niedriger Dispersion, d. h. hoher Abbezahl auf. Dabei liegt die Differenz der beiden Abbezahlen vorzugsweise zwischen 16 und 22. Vorzugsweise sind sowohl die Zerstreuungslinse 21 als auch die Sammellinse 22 aus einem Material mit hoher Brechzahl gefertigt, bevorzugt mit einer Brechzahl größer oder gleich 1,6. Durch Verwendung von Linsen mit hoher Brechzahl lassen sich gleichzeitig notwendige Korrekturen der monochromatischen Bildfehler, wie sphärische Aberration, Koma oder Astigmatismus korrigieren. Eine zusätzliche Korrektur der monochromatischen Bildfehler lässt sich erreichen, wenn die Sammellinse 22 und die Zerstreuungslinse 21 annähernd gleiche Brennweiten, jedoch mit verschiedenen Vorzeichen aufweisen.

Eine sehr gute, kontrastreiche Bildqualität ist erreicht, wenn die Farbbereiche der rechten Teilstrahlen 31, 32, 33 des rechten Beobachtungsstrahlengangs 30, sowie die Farbbereiche der linken Teilstrahlen 41, 42, 43 des linken Beobachtungsstrahlengangs jeweils in der Okular-Zwischenbildebene 35, 45 als deckungsgleich empfunden werden. Dazu ist es notwendig, dass für alle Wellenlängenbereiche des sichtbaren Lichts zwischen 480nm und 660nm ein chromatische Winkelabweichung erreicht ist, die kleiner ist als 0,5'.

Wie in Figur 2 gezeigt, sind die objektseitigen und bildseitigen Brennweiten des optischen Systems bezüglich der beiden Hauptebenen H und H' jeweils abhängig von der Wellenlänge $\lambda$. Dabei sind die folgenden drei Brennweiten von Bedeutung:

fe = objektseitige Brennweite für die Hauptwellenlänge e bezüglich der ersten Hauptebene H;

$f_\lambda$ = objektseitige Brennweite für die Wellenlänge $\lambda$ bezüglich der ersten Hauptebene H;

fe' = bildseitige Brennweite für die Hauptwellenlänge e bezüglich der zweiten Hauptebene H'.

Ist das Linsensystem derart ausgestaltet, d. h. die Reduzieroptik, umfassend die Zerstreuungslinse 21 und die Sammellinse 22, sowie das Hauptobjektiv 20 derart aufeinander abgestimmt, dass für einen Wellenlängenbereich $\lambda$ von 480nm $\leq \lambda \leq$ 660nm und für eine Hauptwellenlänge e = 546nm folgende Beziehung erfüllt ist,

$$\left| \arctan\left( \frac{B}{fe' + (fe \cdot fe'/(f_\lambda - fe))} \right) \right| < 0.5' ,$$

so ist die Abbildungsqualität für einen Beobachtungsstrahlengang so gut korrigiert, dass ein gleichbleibender guter Kontrast der Abbildung und eine gleichbleibende, sehr gute Bildqualität über den gesamten Wellenlängenbereich $\lambda$ erreicht werden. Damit werden die Farbbereiche der rechten Teilstrahlen 31, 32, 33 des rechten Beobachtungsstrahlengangs 30, sowie die Farbbereiche der linken Teilstrahlen 41, 42, 43 des linken Beobachtungsstrahlengangs in der Okular-Zwischenbildebene 35, 45 jeweils als deckungsgleich empfunden. Damit ist eine chromatische Winkelabweichung erreicht, die kleiner ist als 0,5'.

Beispielsweise lässt sich für eine Wellenlänge $\lambda$ = 660nm die chromatische Winkelabweichung CWA für das Ausführungsbeispiel wie folgt berechnen:

$$fe = -175,102mm \ (\text{für } e = 546nm)$$

$$fe' = +175,102mm \ (\text{für } e = 546nm)$$

$$f\lambda = -175,033mm \ (\text{für } \lambda = 660nm)$$

$$B = 12mm$$

Damit ergibt sich für die CWA (in Winkelminuten):

$$CWA = \left| \arctan\left( \frac{B}{fe' + (fe \cdot fe'/(f\lambda - fe))} \right) \right|$$

$$= \left| \arctan\left( \frac{12mm}{175,102mm + (-175,102mm \cdot 175,102mm/(-175,033mm - (-175,102mm)))} \right) \right|$$

$$= 0,093'$$

Da 0,093' < 0,5' ist die Bedingung für eine gute Bildqualität bei einer geringen chromatischen Winkelabweichung CWA für eine Wellenlänge $\lambda$ = 660nm und eine Hauptwellenlänge e = 546nm erfüllt.

Ist diese Bedingung für alle Wellenlängen $\lambda$ zwischen 480nm und 660nm erfüllt, so ist die gewählte Material- und Form-Kombination für die Zerstreuungslinse 21 und die Sammellinse 22 der Reduzieroptik geeignet, die Aufgabe zu erfüllen.

Beim Einbringen der Reduzieroptik in die Beobachtungsstrahlengänge 30, 40 ist es wünschenswert, dass eine Veränderung der Fokuseinstellung des Mikroskops nicht notwendig ist. Dazu ist es von Vorteil, wenn eine Brennweitenänderung, d. h. eine Fokussierung auf die Bildebene 10, durch die Reduzieroptik vorgenommen werden kann. Im Ausführungsbeispiel in Figur 1 ist die gesamte Brennweite $F_{ges}$ des optischen Systems, umfassend das Hauptobjektiv 20 und die Reduzieroptik, so gewählt, dass diese im Bereich zwischen dem 0,7-fachen und dem 1,1-fachen der Brennweite des Hauptobjektivs $F_{HO}$ liegt. Dieser Wert ist ausreichend, um eine Fokussierung durch die Reduzieroptik auf die Bildebene 10 zu ermöglichen. Dazu ist die Zerstreuungslinse 21 fest angeordnet und die Sammellinse 22 verschiebbar entlang der optischen Achse 23 angeordnet, wie durch den Doppelpfeil oberhalb der Sammellinse 22 in Figur 1 angedeutet ist. In einer alternativen Ausführungsform kann auch die Sammellinse 22 fest angeordnet und die Zerstreuungslinse 21 verschiebbar entlang der optischen Achse 23 angeordnet sein. Die Figur 1 zeigt eine mittlere Fokuseinstellung mit einer Brennweite $F_{ges} = 0.87 * F_{HO}$.

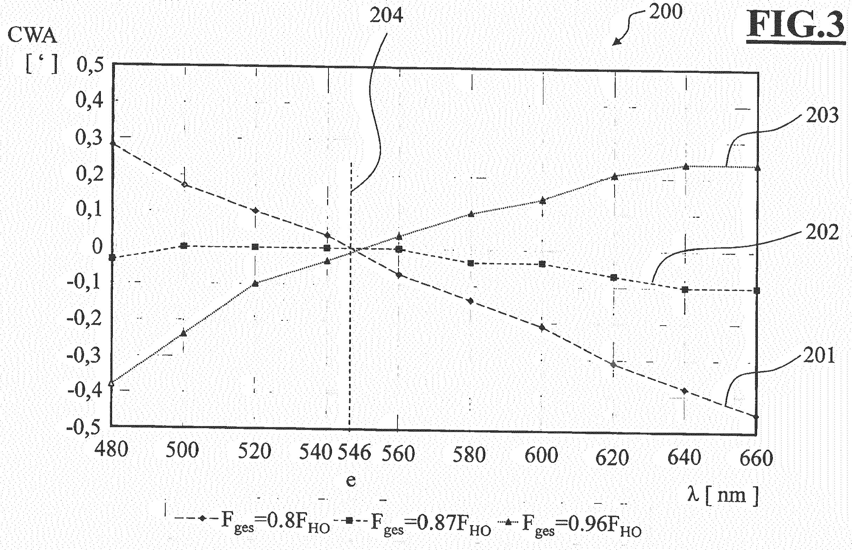

Ein Ergebnis für eine geeignete Material- und Formfestlegung für die Zerstreuungslinse 21 und die Sammellinse 22, die für die Wellenlängen des sichtbaren Lichts und verschiedenen Fokuseinstellungen ein chromatisch korrigiertes, kontrastreiches Bild liefert, zeigt das Diagramm in Figur 3.

In Figur 3 ist eine bildseitige chromatische Winkelabweichung CWA bezogen auf eine Wellenlänge $\lambda$ im sichtbaren Bereich zwischen 480nm und 660nm für drei unterschiedliche Brennweiten des ersten Ausführungsbeispiels dargestellt.

Das Diagramm 200 zeigt die chromatische Winkelabweichung CWA in Winkelminuten auf der Y-Achse in einem Bereich von -0,5' bis +0,5'. Auf der X-Achse ist der Wellenlängenbereich $\lambda$ zwischen 480nm und 660nm dargestellt. Die Hauptwellenlänge e = 546nm ist durch eine gestrichelte Linie 204 hervorgehoben. Die chromatische Winkelabweichung CWA ist für drei Brennweiten dargestellt: Eine erste Brennweite $F_{ges} = 0,80 * F_{HO}$ ist in einer ersten Kurve 201 gezeigt; eine zweite Brennweite $F_{ges} = 0,87 * F_{HO}$ ist in einer zweiten Kurve 202 dargestellt und eine dritte Brennweite $F_{ges} = 0,96 * F_{HO}$ ist in einer dritten Kurve 203 gezeigt. Die erste Kurven 201 und die dritte Kurve 203 zeigen die Fokuseinstellung jeweils in einer möglichen Endlagenposition.

Für die mittlere Fokuseinstellung mit einer Brennweite $F_{ges} = 0,87 * F_{HO}$ ist für den gesamten Wellenlängenbereich $\lambda$ zwischen 480nm und 660nm die bildseitige chromatische Winkelabweichung CWA besonders gut korrigiert und liegt im Bereich zwischen -0,1' und null, siehe zweite Kurve 202. Für die Hauptwellenlänge e = 546nm ist chromatische Winkelabweichung CWA gleich null.

Die chromatische Winkelabweichung CWA für eine Fokuseinstellung mit der Brennweite $F_{ges} = 0,80 * F_{HO}$ liegt für den gesamten Wellenlängenbereich $\lambda$ zwischen 480nm und 660nm im Bereich zwischen +0,28' und -0,45', siehe erste Kurve 201. Auch in dieser Fokuseinstellung ist für die Hauptwellenlänge e = 546nm die chromatische Winkelabweichung CWA gleich null.

Die bildseitige chromatische Winkelabweichung CWA für eine Fokuseinstellung mit der Brennweite $F_{ges} = 0.97 * F_{HO}$ liegt für den gesamten Wellenlängenbereich $\lambda$ zwischen 480nm und 660nm im Bereich zwischen -0,39' und +0,24', siehe dritte Kurve 203. Auch in dieser Fokuseinstellung ist für die Hauptwellenlänge e = 546nm die bildseitige chromatische Winkelabweichung CWA gleich null.

Die Kurven 201, 202 und 203 zeigen deutlich, dass die bildseitige chromatische Winkelabweichung CWA über den gesamten Fokussierbereich für alle Brennweiten im Bereich von +/- 0,5' liegt.

Damit ist ein optisches Abbildungssystem 1 bereitgestellt, das unter Verwendung einer in einen Strahlengang 30, 40 einbringbaren fokussierbaren Reduzieroptik, umfassend eine Zerstreuungslinse 21 und eine Sammellinse 22, eine sehr geringe Baulänge vor einem Hauptobjektiv erreicht. Dieses optische Abbildungssystem erreicht eine sehr gute Abbildungsqualität bei einer äußerst geringen chromatischen Winkelabweichung über den gesamten Fokussierbereich.

**Bezugszeichenliste**

| | |
|---|---|
| 1 | Optisches Abbildungssystem |
| 2 | Auge |
| 3 | Ophthalmoskopierlupe |
| 10 | Bildebene |
| 20 | Hauptobjektiv |
| 21 | Zerstreuungslinse |
| 22 | Sammellinse |
| 23 | Optische Achse |
| 30 | Rechter Beobachtungsstrahlengang |
| 31 | Rechter roter Teilstrahl |
| 32 | Rechter grüner Teilstrahl |

33    Rechter blauer Teilstrahl
34    Rechte Tubuslinse
35    Rechte Okular-Zwischenbildebene
40    Linker Beobachtungsstrahlengang
41    Linker roter Teilstrahl
42    Linker grüner Teilstrahl
43    Linker blauer Teilstrahl
44    Linke Tubuslinse
45    Linke Okular-Zwischenbildebene
101    Erster Strahl des Strahlengangs vom Objektpunkt O zu Hauptebene H im Objektraum
102    Zweiter Strahl des Strahlengangs zwischen den Hauptebenen H und H'
103    Dritter Strahl des Strahlengangs von der Hauptebene H' zum Bildpunkt O' im Bildraum
200    Diagramm chromatische Winkelabweichung CWA
201    Erste Kurve, CWA für $F_{ges}= 0.80F_{HO}$
202    Zweite Kurve, CWA für $F_{ges}= 0.87F_{HO}$
203    Dritte Kurve, CWA für $F_{ges}= 0.96F_{HO}$

**Patentansprüche**

1.    Optisches Abbildungssystem (1) zur Erzeugung eines Bildes einer Objektebene mit einem Linsensystem, das ein Hauptobjektiv (20) und eine Reduzieroptik zwischen dem Hauptobjektiv (20) und der Objektebene umfasst und das entlang einer optischen Achse (23) des Linsensystems ausgerichtet ist, wobei die Reduzieroptik eine erste Linse (22) mit einer positiven Brechkraft und eine zweite Linse (21) mit einer negativen Brechkraft aufweist,
       wobei eine objektseitige erste Hauptebene (H) und eine bildseitige zweite Hauptebene (H') durch das Linsensystem definiert ist,
       wobei durch das optische Abbildungssystem (1) ein erster Beobachtungsstrahlengang (30) und ein zweiter Beobachtungsstrahlengang (40) definiert ist, die derart durch das Linsensystem geführt sind, dass die Beobachtungsstrahlengänge (30, 40) in der ersten Hauptebene (H) und in der zweiten Hauptebene (H') jeweils einen Abstand B zur optischen Achse (23) des Linsensystems aufweisen,
       **dadurch kennzeichnet, dass**
       die erste Linse (22) aus einem ersten Material gefertigt ist, das eine erste Abbezahl aufweist, und dass die zweite Linse (21) aus einem zweiten Material gefertigt ist, das eine zweite Abbezahl aufweist, wobei die erste Abbezahl größer ist als die zweite Abbezahl,
       und dass das Linsensystem derart ausgestaltet ist, dass für alle Wellenlängen im Wellenlängenbereich $\lambda$ von 480nm $\leq$ $\lambda$ $\leq$ 660nm und für eine Hauptwellenlänge e = 546nm folgende Beziehung erfüllt ist:

$$\left|\arctan\left(\frac{B}{f_e' + (f_e \cdot f_e' / (f_\lambda - f_e))}\right)\right| < 0.5', \text{ in Winkelminuten wobei gilt:}$$

       $f_e$ = objektseitige Brennweite für die Hauptwellenlänge e bezüglich der ersten Hauptebene (H);
       $f_\lambda$ = objektseitige Brennweite für die Wellenlänge $\lambda$ bezüglich der ersten Hauptebene (H);
       $f_e'$ = bildseitige Brennweite für die Hauptwellenlänge e bezüglich der zweiten Hauptebene (H').

2.    Optisches Abbildungssystem nach Anspruch 1,
       **dadurch kennzeichnet, dass**
       das erste Material und das zweite Material derart gewählt sind, dass eine Differenz der ersten Abbezahl und der zweiten Abbezahl zwischen 16 und 22 liegt.

3.    Optisches Abbildungssystem nach Anspruch 1 oder 2,
       **dadurch kennzeichnet, dass**
       das erste Material und das zweite Material derart gewählt sind, dass eine erste Brechzahl des ersten Materials größer ist als 1,6 und eine zweite Brechzahl des zweiten Materials größer ist als 1,6.

4.    Optisches Abbildungssystem nach einem der Ansprüche 1 bis 3,
       **dadurch kennzeichnet, dass**
       die erste Linse (22) fest angeordnet ist und die zweite Linse (21) verschiebbar in Richtung der optischen Achse angeordnet ist.

**5.** Optisches Abbildungssystem nach einem der Ansprüche 1 bis 3,
**dadurch kennzeichnet, dass**
die zweite Linse (21) fest angeordnet ist und die erste Linse (22) verschiebbar in Richtung der optischen Achse angeordnet ist.

**6.** Optisches Abbildungssystem nach einem der vorherigen Ansprüche,
**dadurch kennzeichnet, dass**
dass die Reduzieroptik in den Strahlengang vor dem Hauptobjektiv (20) einschwenkbar ist.

**7.** Optisches Abbildungssystem nach einem der vorherigen Ansprüche,
**dadurch kennzeichnet, dass**
das vor der Reduzieroptik ein weiteres optisches Element (3) zur Erzeugung eines Zwischenbildes (10) im Beobachtungsstrahlengang angebracht ist und dass das optische Abbildungssystem (1) auf das Zwischenbild (10) fokussiert ist.

**8.** Optisches Abbildungssystem nach einem der vorherigen Ansprüche,
**dadurch kennzeichnet, dass**
das optische Abbildungssystem (1) als ein Stereomikroskop ausgebildet ist

**Claims**

**1.** Optical imaging system (1) for generating an image of an object plane, comprising a lens system which comprises a main objective (20) and a reduction optical unit between the main objective (20) and the object plane and which is aligned along an optical axis (23) of the lens system,
wherein the reduction optical unit comprises a first lens (22) with a positive refractive power and a second lens (21) with a negative refractive power,
wherein an object-side first main plane (H) and an image-side second main plane (H') are defined by the lens system,
wherein the optical imaging system (1) defines a first observation beam path (30) and a second observation beam path (40) which are guided through the lens system in such a way that, in the first main plane (H) and in the second main plane (H'), the observation beam paths (30, 40) in each case have a distance B from the optical axis (23) of the lens system,
**characterized in that**
the first lens (22) is manufactured from a first material which has a first Abbe number, and **in that** the second lens (21) is manufactured from a second material which has a second Abbe number, wherein the first Abbe number is greater than the second Abbe number,
and **in that** the lens system is configured in such a way that the following relation is satisfied for all wavelengths in the wavelength range $\lambda$ of 480 nm$\leq\lambda\leq$660 nm and for a main wavelength e=546 nm:

$$\left|\arctan\left(\frac{B}{f_e'+\left(f_e \cdot f_e'/\left(f_\lambda - f_e\right)\right)}\right)\right| < 0.5',$$ in angular minutes where:

$f_e$ = object-side focal length for the main wavelength e in respect of the first main plane (H);
$f_\lambda$ = object-side focal length for the wavelength $\lambda$ in respect of the first main plane (H);
$f_e'$ = image-side focal length for the main wavelength e in respect of the second main plane (H').

**2.** Optical imaging system according to Claim 1,
**characterized in that**
the first material and the second material are selected in such a way that a difference between the first Abbe number and the second Abbe number lies between 16 and 22.

**3.** Optical imaging system according to Claim 1 or 2,
**characterized in that**
the first material and the second material are selected in such a way that a first refractive index of the first material is greater than 1.6 and a second refractive index of the second material is greater than 1.6.

**4.** Optical imaging system according to one of Claims 1 to 3,

**characterized in that**

the first lens (22) is disposed in a stationary manner and the second lens (21) is disposed displaceably in the direction of the optical axis.

5. Optical imaging system according to one of Claims 1 to 3,
**characterized in that**
the second lens (21) is disposed in a stationary manner and the first lens (22) is disposed displaceably in the direction of the optical axis.

6. Optical imaging system according to one of the preceding claims,
**characterized in that**
the reduction optical unit can be pivoted into the beam path upstream of the main objective (20).

7. Optical imaging system according to one of the preceding claims,
**characterized in that**
a further optical element (3) for generating an intermediate image (10) is fastened in the observation beam path upstream of the reduction optical unit and **in that** the optical imaging system (1) is focused onto the intermediate image (10) .

8. Optical imaging system according to one of the preceding claims,
**characterized in that**
the optical imaging system (1) is embodied as a stereo microscope.

**Revendications**

1. Système d'imagerie optique (1) destiné à l'obtention d'une image d'un plan d'objet avec un système de lentilles, lequel comprend un objectif principal (20) et une optique réductrice entre l'objectif principal (20) et le plan d'objet et est orienté le long d'un axe optique (23) du système de lentilles,
dans lequel l'optique réductrice comporte une première lentille (22) avec une puissance optique positive et une deuxième lentille (21) avec une puissance optique négative,
dans lequel sont définis un premier plan principal (H) côté objet et un deuxième plan principal (H') côté image par le biais du système de lentilles,
dans lequel sont définis par le biais du système d'imagerie optique (1) un premier faisceau d'observation (30) et un deuxième faisceau d'observation (40), lesquels sont guidés au travers du système de lentilles de telle sorte que les faisceaux d'observation (30, 40) possèdent chacun un écart B par rapport à l'axe optique (23) du système de lentilles dans le premier plan principal (H) et dans le deuxième plan principal (H'),
**caractérisé en ce que** la première lentille (22) est fabriquée à partir d'un premier matériau possédant un premier nombre d'Abbe, et **en ce que** la deuxième lentille (21) est fabriquée à partir d'un deuxième matériau possédant un deuxième nombre d'Abbe, dans lequel le premier nombre d'Abbe est supérieur au deuxième nombre d'Abbe,
et **en ce que** le système de lentilles est disposé de telle sorte que la relation suivante est vraie pour toutes les longueurs d'onde dans un intervalle de longueur d'onde $\lambda$ de 480 nm $\leq \lambda \leq$ 660 nm et pour une longueur d'onde principale e = 546 nm : $\left| \arctan\left( \dfrac{B}{f_e' + \left(f_e \cdot f_e'/(f_\lambda - f_e)\right)} \right) \right| < 0.5'$, en minutes d'angle dans

laquelle :

$f_e$ = distance focale côté objet pour la longueur d'onde principale e par rapport au premier plan principal (H) ;
$f_\lambda$ = distance focale côté objet pour la longueur d'onde
$\lambda$ par rapport au premier plan principal (H) ;
$f_e'$ = distance focale côté image pour la longueur d'onde principale e par rapport au deuxième plan principal (H') ;

2. Système d'imagerie optique selon la revendication 1, **caractérisé en ce que** le premier matériau et le deuxième matériau sont choisis de telle sorte qu'une différence entre le premier nombre d'Abbe et le deuxième nombre d'Abbe est comprise entre 16 et 22.

3. Système d'imagerie optique selon la revendication 1 ou 2, **caractérisé en ce que** le premier matériau et le deuxième matériau sont choisis de telle sorte qu'un premier indice de réfraction du premier matériau est supérieur à 1,6 et un deuxième indice de réfraction du deuxième matériau est supérieur à 1,6.

4. Système d'imagerie optique selon l'une des revendications 1 à 3, **caractérisé en ce que** la première lentille (22) est agencée de manière fixe et la deuxième lentille (21) est agencée de manière mobile dans la direction de l'axe optique.

5. Système d'imagerie optique selon l'une des revendications 1 à 3, **caractérisé en ce que** la deuxième lentille (21) est agencée de manière fixe et la première lentille (22) est agencée de manière mobile dans la direction de l'axe optique.

6. Système d'imagerie optique selon l'une des revendications ci-dessus, **caractérisé en ce que** l'optique réductrice peut être pivotée dans le faisceau devant l'objectif principal (20).

7. Système d'imagerie optique selon l'une des revendications ci-dessus, **caractérisé en ce qu'**un élément optique supplémentaire (3) est agencé devant l'optique réductrice afin d'obtenir une image intermédiaire (10) dans le faisceau d'observation et **en ce que** le système d'imagerie optique (1) est focalisé sur l'image intermédiaire (10).

8. Système d'imagerie optique selon l'une des revendications ci-dessus, **caractérisé en ce que** le système d'imagerie optique (1) est conçu comme un microscope stéréoscopique.

**FIG.1**

FIG.2

EP 2 853 933 B1

FIG.3

CWA
[ ' ]

λ [ nm ]
e

$- \blacklozenge -F_{ges}=0.8F_{HO}$  $- \blacksquare -F_{ges}=0.87F_{HO}$  $\cdots \blacktriangle \cdots F_{ges}=0.96F_{HO}$

EP 2 853 933 B1

**IN DER BESCHREIBUNG AUFGEFÜHRTE DOKUMENTE**

**In der Beschreibung aufgeführte Patentdokumente**

- EP 1227355 B1
- EP 1326117 A1
- CH 663284 A5
- DE 102010018123 A1